# EUROPEAN PATENT SPECIFICATION

(11) **EP 1 448 095 B1**
(45) Date of publication and mention of the grant of the patent: **11.10.2006**
(21) Application number: 02777719.2
(22) Date of filing: 30.10.2002
(51) Int. Cl.: A61B 5/055, A61B 6/04

(54) **MEDICAL IMAGING SYSTEM**
MEDIZINISCHES BILDERZEUGUNGSSYSTEM
SYSTEME D'IMAGERIE MEDICALE

(30) Priority: 14.11.2001 EP 01204347
(43) Date of publication of application: 25.08.2004
(73) Proprietor: Koninklijke Philips Electronics N.V., 5621 BA Eindhoven (NL)
(72) Inventor: SEIJGER, Olav, J., NL-5656 AA Eindhoven (NL); LENTING, Gerrit, J., NL-5656 AA Eindhoven (NL)
(74) Representative: Wolfs, Marc Johannes Maria
(86) International application number: PCT/IB2002/004572
(87) International publication number: WO 2003/041578

(56) References cited:
- EP-A- 0 712 606
- EP-A- 0 995 397
- WO-A-02/062219
- PATENT ABSTRACTS OF JAPAN vol. 1999, no. 05, 31 May 1999 (1999-05-31) -& JP 11 033010 A (HITACHI MEDICAL CORP), 9 February 1999 (1999-02-09)
- PATENT ABSTRACTS OF JAPAN vol. 2000, no. 23, 10 February 2001 (2001-02-10) -& JP 2001 170021 A (HITACHI MEDICAL CORP), 26 June 2001 (2001-06-26)

## Description

The invention relates to a medical imaging system comprising a frame accommodating an imaging device and an examination volume, the system further comprising a patient support member, a first displacement device for displacing the patient support member relative to the frame in a first horizontal direction into and out of the examination volume, and a second displacement device for displacing the patient support member relative to the frame in a second horizontal direction transverse to the first horizontal direction, the patient support member being provided on a mobile carrier which comprises a first docking member by means of which the carrier can be docked to a second docking member provided on the frame.

Medical imaging systems of the kind mentioned in the opening paragraph are generally known. An example of a known medical imaging system is a magnetic resonance imaging system by means of which images can be made of the entrails of a patient using nuclear magnetic resonance methods. The imaging device of the known magnetic resonance imaging system comprises an electrical coil system for generating a varying magnetic field and radio-frequency signals in the examination volume, in which the patient is to be placed, and for receiving radio-frequency signals generated by the patient's body in response to the radio-frequency signals generated by the coil system. The patient support member is a horizontal bed, and the mobile carrier, on which the patient support member is provided, is a trolley. Since the patient support member is provided on the mobile carrier, the patient support member with the patient lying on it can be moved from the imaging system to, for example, a preparation room or to another medical examination or treatment device. By means of the docking members, the mobile carrier can be mechanically coupled to the frame, so that the patient support member provided on the mobile carrier is brought in a stable position relative to the frame and the imaging device. The docking members also comprise electrical connections between the mobile carrier and the imaging device, so that a battery, which is provided on the mobile carrier for feeding electrical systems of the mobile carrier, can be charged and information about the patient, stored in an electrical memory of the mobile carrier, can be exchanged between the mobile carrier and the imaging device. The first displacement device is provided in the mobile carrier. In the docked condition of the mobile carrier, the patient support member with the patient lying on it can be displaced by the first displacement device relative to the mobile carrier and relative to the frame, to which the mobile carrier is docked, in the first horizontal direction, so that the patient is moved into the examination volume and, after the examination, out of the examination volume. The second displacement device is also provided in the mobile carrier, so that, in the docked condition of the mobile carrier, the patient can also be moved relative to the frame and the imaging device in the second horizontal direction. This is often necessary to bring the part of the patient's body to be examined in a correct position relative to the imaging device.

A drawback of the known medical imaging system is that, in particular in the case of corpulent patients, the center of gravity of the patient support member and the patient lying on it comes close to the wheels of the mobile carrier, seen in the second horizontal direction, when the patient support member with the patient lying on it is displaced relative to the mobile carrier in the second horizontal direction, whereby the stability of the mobile carrier strongly decreases and the mobile carrier could fall over. As a result, the distance over which the patient support member can be displaced in the second direction is limited.

JP-A-11 033010 describes a patient bed 52 with a top plate which is movable in the longitudinal direction along a side frame 53 - see arrow Y. The bed 52 has four legs 56 with casters 57. A slide guide 62 is arranged in a mounting metal fitting 61 of a static magnetic field generating device 51 and a plate 60 mounted to the bed 52 is fixed to the slide guide 62 by means of a screw 63. Thus, the bed can be moved longitudinally towards the device 51 and the bed can be moved perpendicularly in direction of the arrow X by means of the slide guide 62.

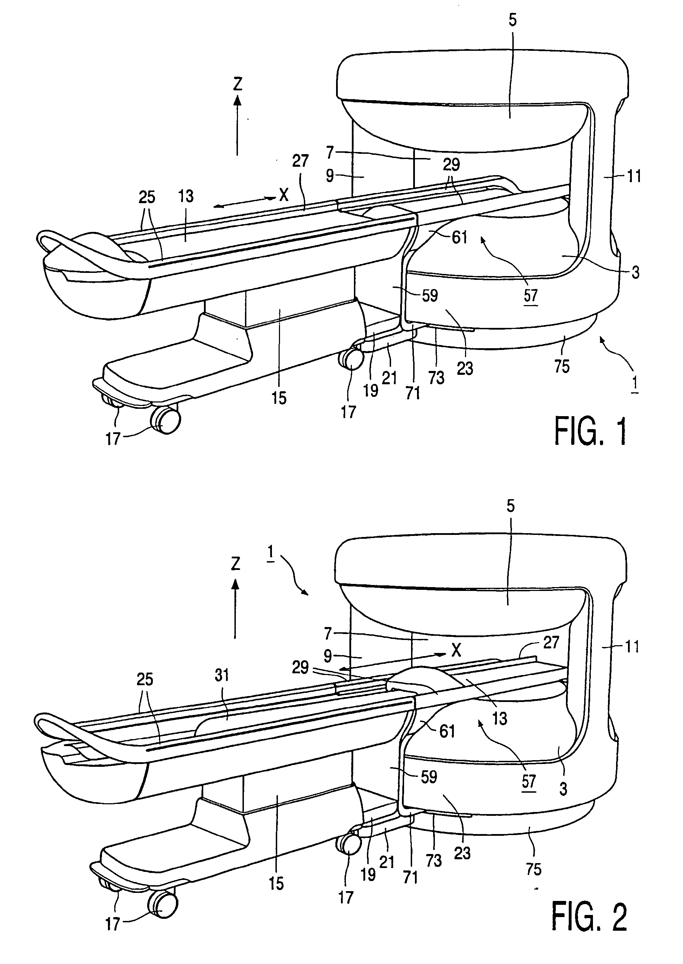

JP-A-2001 1170021 describes a bed 15 with a top board 19 having four legs 17 with wheels 16. An elevator mechanism is provided for raising/lowering the top board 19 of the bed 15. Further a connecting mechanism 21 is mounted to the base 11 of a diagnostic instrument with a yoke 12 and opposing magnets 13. The connecting mechanism is provided for moving the bed 15 in/out of the imaging region of the device (see Fig. 2).

The patient beds on a mobile carrier as described in these documents have similar drawbacks as mentioned above.

An object of the invention is to provide a medical imaging system of the kind mentioned in the opening paragraph which does not have the drawback of the known medical imaging system.

To achieve this object, a medical imaging system according to the invention is characterized in that the second displacement device is provided on the frame for displacing the second docking member relative to the frame in the second horizontal direction. In a medical imaging system according to the invention displacements of the patient support member relative to the frame in the second horizontal direction are generated in the docked condition of the mobile carrier by displacing the second docking member, together with the mobile carrier and the patient support member mechanically coupled thereto, in the second horizontal direction by means of the second displacement device. As a result, the patient support member does not need to be displaced relative to the mobile carrier in the second horizontal direction and can be mounted or maintained in a fixed stable position relative to the mobile carrier, seen in the second horizontal direction. As a result, the patient support member can be displaced in a stable manner in the second horizontal direction over relatively large distances. An additional advantage of a medical imaging system according to the invention, in particular of such systems which comprise at least two mobile carriers each with a separate patient support member which can alternately be docked to the frame, is that the mobile carrier has a relatively simple structure, because the mobile carrier does not accommodate the second displacement device.

A particular embodiment of a medical imaging system according to the invention is characterized in that the carrier is mobile by means of rolling members. The rolling members provide a stable support for the mobile carrier and the patient support member provided thereon when forces are exerted on the mobile carrier via the docking members to displace the mobile carrier in the second horizontal direction.

A further embodiment of a medical imaging system according to the invention is characterized in that the system is a magnetic resonance imaging system and the frame comprises a lower coil system housing and an upper coil system housing between which the examination volume is present, the second docking member and the second displacement device being provided in the lower coil system housing. In this further embodiment, the medical imaging system is a magnetic resonance system of the so-called open type, wherein the examination volume is bounded in the vertical direction by the lower coil system housing and the upper coil system housing and wherein the examination volume is open in the major part of its horizontal plane. The invention is particularly suitable for use in a magnetic resonance imaging device of the open type, because the open examination volume allows relatively large displacements of the patient within the examination volume in the second horizontal direction. As a result, the patient's body can be brought in a more correct position relative to the imaging device than in a magnetic resonance imaging device of the so-called closed type having a relatively narrow cylindrical examination volume. The second docking member and the second displacement device are provided in the lower coil system housing, because the lower coil system is relatively close to the mobile carrier.

A yet further embodiment of a medical imaging system according to the invention is characterized in that the system comprises a guide member which is present in the examination volume, is mounted to the second docking member, and extends in the first horizontal direction for guiding and supporting the patient support member in the examination volume. When the patient support member is displaced in the first horizontal direction into the examination volume, the guiding and supporting functions of the mobile carrier are taken over by the guide member. Since the guide member is mounted to the second docking member, the guide member is displaced by the second docking member in the second horizontal direction together with the mobile carrier docked to the second docking member, so that the guide member is always maintained in a correct position relative to the mobile carrier, i.e. in a position wherein the guide member is in line with a further guide member for the patient support member present on the mobile carrier. As a result, the patient support member can be docked to the second docking member and subsequently be displaced into the examination volume in each possible position of the second docking member, seen in the second horizontal direction, and the patient support member can also be displaced out of the examination volume and subsequently be undocked from the second docking member in each possible position of the patient support member, seen in the second horizontal direction.

A particular embodiment of a medical imaging system according to the invention is characterized in that the guide member is provided on a bridge-shaped further carrier which bridges over the lower coil system housing and comprises a first leg, which is mounted to and supported by the second docking member, and a second leg, which is supported by rolling members at a side of the lower coil system housing opposite to a side where the second docking member is present. The bridge-shaped further carrier provides a practical and stable support for the guide member in the examination volume above the lower coil system housing.

A further embodiment of a medical imaging system according to the invention is characterized in that the guide member is provided on a bridge-shaped further carrier which spans over the lower coil system housing and comprises a first leg, which is mounted to and supported by the second docking member, and a second leg, which is present at a side of the lower coil system housing opposite to a side where the second docking member is present and which is connected to the first leg by means of a connecting member extending underneath the lower coil system housing. The connecting member strongly improves the rigidity of the bridge-shaped further carrier.

Hereafter, embodiments of a medical imaging system according to the invention will be described as shown in the drawings, wherein
Fig. 1 shows a medical imaging system according to the invention,
Fig. 2 shows the medical imaging system of Fig. 1 with a patient support member of the system being present in an examination volume of the system,
Fig. 3 shows a front view of the medical imaging system of Fig. 1,
Fig. 4 schematically shows a displacement device of the medical imaging system of Fig. 1,
Fig. 5 schematically shows a bridge-shaped carrier of the medical imaging system of Fig. 1, and
Fig. 6 schematically shows a bridge-shaped carrier of an alternative embodiment of a medical imaging system according to the invention.

The medical imaging system according to the invention shown in Fig. 1 is a magnetic resonance imaging system of the so-called open type by means of which images can be made of the entrails of a patient using a nuclear magnetic resonance method. The medical imaging system comprises a frame 1 having a lower coil system housing 3 and an upper coil system housing 5 which accommodate an electrical coil system. The coil system, which is not shown in Fig. 1, is a part of an imaging device of the medical imaging system, which is also not shown in Fig. 1 and which can be of a kind which is known per se. Between the lower coil system housing 3 and the upper coil system housing 5, an examination volume 7 is present in which the patient is to be placed. The examination volume 7 is bounded in the vertical direction Z by the lower coil system housing 3 and the upper coil system housing 5. The lower coil system housing 3 and the upper coil system housing 5 are interconnected by means of two vertical columns 9, 11 of the frame 1, which have a relatively small cross-section, so that the examination volume 7 is open in the major part of its horizontal plane. During operation, the coil system generates a varying magnetic field and radio-frequency signals in the examination volume 7. In response to the radio-frequency signals generated by the coil system, the patient's body generates radio-frequency signals, which are received by the coil system and are processed into an image of the patient's body by the imaging device.

The medical imaging system further comprises a patient support member 13, in particular a horizontal bed, which is provided on a mobile carrier 15, in particular a trolley which is mobile by means of rolling members 17. The mobile carrier 15 is provided with a first docking member 19 by means of which the mobile carrier 15 can be docked to a second docking member 21 which is provided on the frame 1, in particular at a front side 23 of the lower coil system housing 3. Since the patient support member 13 is provided on the mobile carrier 15, the patient support member 13 with the patient lying on it can be moved from the medical imaging system to, for example, a separate preparation room, where the patient is prepared before the examination, or to another medical examination or treatment device to undergo a subsequent examination or treatment. Preferably, the medical imaging system comprises at least two mobile carriers 15 each with a separate patient support member 13. In this manner, a second patient can be prepared during the examination of a first patient and, after the examination of the first patient, the mobile carrier 15 with the patient support member 13 carrying the first patient can be undocked from and moved away from the medical imaging system, while the mobile carrier 15 with the patient support member 13 carrying the second patient can immediately be moved to and docked to the medical imaging system. As a result, the medical imaging system can efficiently be used.

By means of the first docking member 19 and the second docking member 21, which are only schematically shown in Fig. 1, the mobile carrier 15 can be mechanically coupled to and uncoupled from the lower coil system housing 3, so that the patient support member 13 provided on the mobile carrier 15 can be brought into a pre-defined stable position relative to the frame 1 and the imaging device. For this purpose, the first docking member 19 and the second docking member 21 can be provided with conventional mechanical coupling means which will not be described further. Preferably, the first docking member 19 and the second docking member 21 also comprise electrical connections between the mobile carrier 15 and the imaging device. In this manner, the mobile carrier 15 or the patient support member 13 can be provided, for example, with an electrical memory in which information about the patient can be stored, which information can be exchanged between said memory and the imaging device via said electrical connections. Furthermore, a battery, which may be provided on the mobile carrier 15 for feeding electrical systems of the mobile carrier 15 or the patient support member 13, can be charged via said electrical connections.

In the docked condition of the mobile carrier 15 shown in Fig. 1, the patient support member 13 with the patient lying on it can be displaced relative to the mobile carrier 15 and the frame 1 in a first horizontal direction X from a first position shown in Fig. 1, in which the patient support member 13 is fully supported by the mobile carrier 15, to a second position shown in Fig. 2, in which the patient support member 13 is present in the examination volume 7. For this purpose, the mobile carrier 15 is provided with a first guide member 25 which, in the docked condition of the mobile carrier 15, extends in the first horizontal direction X and co-operates with guide elements 27 provided on the patient support member 13. The frame 1 is provided with a second guide member 29, which is present in the examination volume 7 and extends in the first horizontal direction X and which, in the docked condition of the mobile carrier 15, is in line with the first guide member 25. The mobile carrier 15 further comprises a first displacement device for displacing the patient support member 13 along the first guide member 25 and along the second guide member 29 into the examination volume 7 and, after the examination of the patient, out of the examination volume 7 again. The first displacement device may be of a conventional kind and may be provided, for example, with an elongate push-pull member 31, shown in Fig. 2, which is fastened to the patient support member 13 and is driven by an electrical motor provided in the mobile carrier 15. When the patient support member 13 is displaced by the first displacement device into the examination volume 7, the guiding and supporting functions of the first guide member 25 of the mobile carrier 15 are taken over by the second guide member 29 and, when the patient support member 13 is displaced out of the examination volume 7, the guiding and supporting functions of the second guide member 29 are taken over again by the first guide member 25.

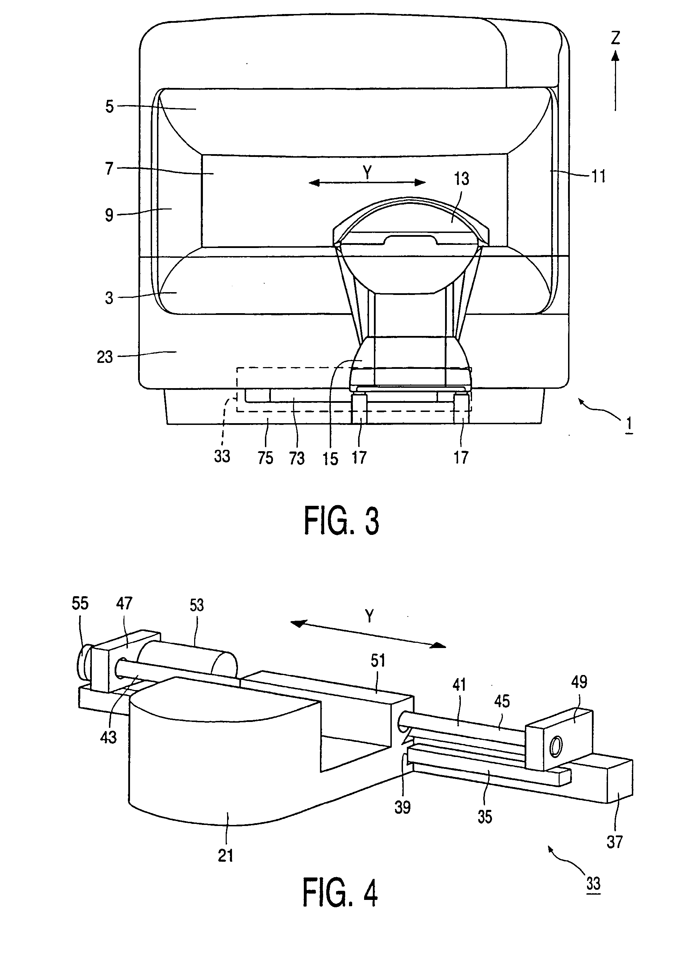

As shown in Fig. 3, the medical imaging system further comprises a second displacement device 33 by means of which the patient support member 13, in the docked condition of the mobile carrier 15, can be displaced relative to the frame 1 and the imaging device in a second horizontal direction Y transverse to the first horizontal direction X. By displacing the patient relative to the imaging device in the second horizontal direction Y, the portion of the patient's body to be examined can be brought into a correct position relative to the imaging device. As shown in Fig. 3, relatively large displacements of the patient support member 13 in the second horizontal direction Y are possible as a result of the fact that the examination volume 7 is open in the major part of its horizontal plane. As a result, the patient's body can be brought into a more correct position relative to the imaging device than in a magnetic resonance imaging device of the so-called closed type, which has a relatively narrow cylindrical examination volume.

In order to allow the patient support member 13 to be displaced in the second horizontal direction Y relative to the frame 1 and the imaging device in a stable manner and over relatively large distances, the second displacement device 33 is provided on the frame 1, in particular in the lower coil system housing 3, for displacing the second docking member 21 relative to the frame 1 in the second horizontal direction Y. Fig. 3 only schematically shows the position of the second displacement device 33 in the lower coil system housing 3 by means of broken lines. Fig. 4 schematically shows the main parts of the second displacement device 33. The second displacement device 33 comprises a linear guide member 35, which extends in the second horizontal direction Y and which is mounted to a frame member 37 which is fixed to the lower coil system housing 3. The second docking member 21, which is only schematically shown in Fig. 4, comprises a guide element 39 for co-operation with the linear guide member 35. The second displacement device 33 further comprises a spindle 41, which also extends in the second horizontal direction Y and which is provided with a screw-thread nut shown in Fig. 4. A first end portion 43 and a second end portion 45 of the spindle 41 are journalled respectively in a first bearing bush 47 and in a second bearing bush 49, which are both mounted to the frame member 37. A third bearing bush 51, which comprises an internal screw thread for co-operation with the screw-thread of the spindle 41, is mounted to the second docking member 21. The second displacement device 33 further comprises an electrical motor 53 mounted to the frame member 37 for driving a gear wheel 55 which is mounted to the first end portion 43 of the spindle 41. When the gear wheel 55 is driven by the motor 53, the second docking member 21 is displaced along the linear guide member 35 as a result of the co-operation between the screw-threads of the spindle 41 and of the third bearing bush 51.

With the above constitution of the second displacement device 33, displacements of the patient support member 13 relative to the frame 1 in the second horizontal direction Y are generated in the docked condition of the mobile carrier 15 by displacing the second docking member 21, together with the mobile carrier 15 and the patient support member 13 mechanically coupled to the second docking member 21, in the second horizontal direction Y by means of the second displacement device 33. As a result, the mobile carrier 15 does not need to be provided with an additional displacement device for displacing the patient support member 13 in the second horizontal direction Y relative to the mobile carrier 15, so that the patient support member 13 can be mounted and maintained, seen in the second horizontal direction Y, in a fixed central position relative to the mobile carrier 15. As a result, the patient's center of gravity is always maintained, seen in the second horizontal direction Y, in a central position relative to the rolling members 17 during displacements of the patient support member 13 in the second horizontal direction Y, so that the patient support member 13 is displaced in a stable manner in the second horizontal direction Y relative to the frame 1. The maximum distance over which the patient support member 13 can be displaced relative to the frame 1 in the second horizontal direction Y is not limited by the dimensions of the mobile carrier 15 in the second horizontal direction Y, but is determined by the length of the spindle 41, which can be such that the patient support member 13 can be displaced relative to the frame 1 in the Y-direction over the relatively large distance allowed by the dimensions of the examination volume 7. The rolling members 17 provide a stable support for the mobile carrier 15 and the patient support member 13 during displacements of the mobile carrier 15 in the second horizontal direction Y by means of the second displacement device 33. The stability of the mobile carrier 15 during displacements in the second horizontal direction Y is further improved by the fact that the first docking member 19 and the second docking member 21 are provided, to the extent possible, near the floor surface, i.e. to the extent possible near the imaginary horizontal surface in which the rolling member 17 are situated. As a result, mechanical moments resulting from the forces exerted on the mobile carrier 15 via the docking members 19 and 21 during displacements of the mobile carrier 15 by the second displacement device 33 and causing instability of the mobile carrier 15 are limited as much as possible. Since the second displacement device 33 is provided on the frame 1 and not on the mobile carrier 15, the structure of the mobile carrier 15 is relatively simple. This is particularly advantageous in embodiments of a medical imaging system according to the invention which are provided with two or more than two mobile carriers 15, each with a separate patient support member 13, which can alternately be docked to the frame 1.

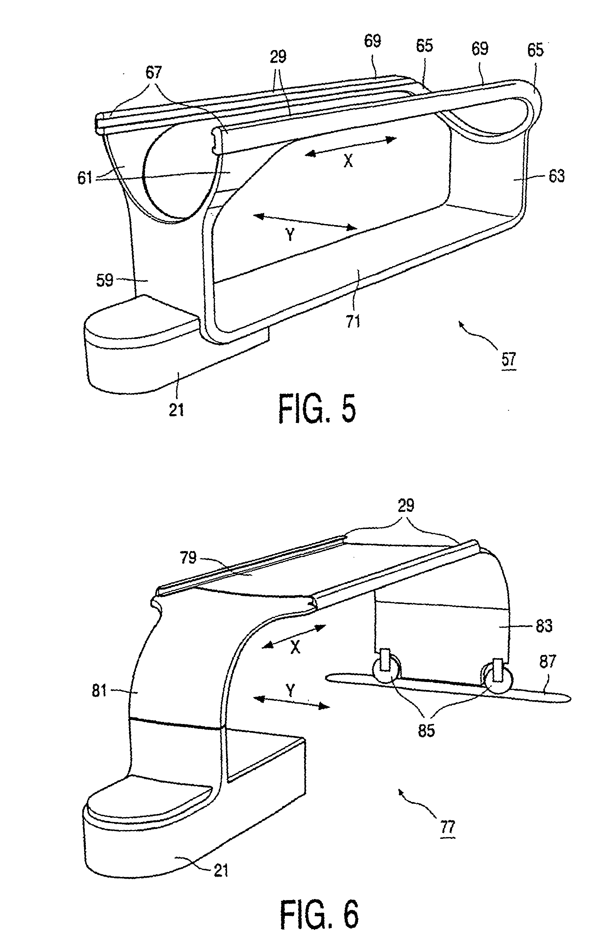

As shown in Fig. 1, the second guide member 29, which is present in the examination volume 7 for guiding and supporting the patient support member 13 in the examination volume 7, is mounted on a further carrier 57 which is bridge-shaped and spans the lower coil system housing 3. The bridge-shaped further carrier 57, which is separately shown in Fig. 5, comprises a first substantially vertical leg 59, which is situated at the front side 23 of the lower coil system housing 3 and comprises curved supporting members 61, and a second substantially vertical leg 63, which is situated at a back side of the lower coil system housing 3 opposite to the front side 23 and comprises curved supporting members 65. A first end portion 67 and a second end portion 69 of the second guide member 29 are mounted to respectively the curved supporting members 61 and the curved supporting members 65. The lower end portion of the first leg 59 is connected to the lower end portion of the second leg 63 by means of a horizontal plate-shaped connecting member 71 which extends underneath the lower coil system housing 3 in a gap-shaped opening 73, which is present between the lower coil system housing 3 and a foot 75 of the frame 1 as shown in Fig. 3. Thus, the bridge-shaped further carrier 57 has a relatively high rigidity.

As shown in Fig. 1 and Fig. 5, the first leg 59 is mounted to and supported by the second docking member 21, which is only schematically shown in Fig. 5. As a result, the bridge-shaped further carrier 57 and the second guide member 29 are arranged in a fixed position relative to the second docking member 21, so that the second guide member 29 is displaced by the second displacement device 33 in the second horizontal direction Y together with the mobile carrier 15 and the patient support member 13 docked to the second docking member 21. As a result, the second guide member 29 is maintained in a correct position relative to the mobile carrier 15 during displacements of the mobile carrier 15 in the second horizontal direction Y, i.e. in a position wherein the second guide member 29 is in line with the first guide member 25 provided on the mobile carrier 15. Furthermore, the first guide member 25 is always brought into a correct position in line with the second guide member 29, i.e. irrespective of the position of the second docking member 21 in the second horizontal direction Y, when the mobile carrier 15 is moved to the frame 1 and docked thereto. Consequently, the mobile carrier 15 can be docked to the second docking member 21 and the patient support member 13 can subsequently be displaced into the examination volume 7 in each possible position of the second docking member 21, seen in the second horizontal direction Y. Furthermore, the patient support member 13 can be displaced out of the examination volume 7 and the mobile carrier 15 can subsequently be undocked from the second docking member 21 in each possible position of the patient support member 13 in the examination volume 7, seen in the second horizontal direction Y. It is noted, that the plate-shaped connecting member 71 can be provided with means, such as rolling members, for supporting and guiding the connecting member 71 in the gap-shaped opening 73.

Fig. 6 shows a bridge-shaped carrier 77 carrying the second guide member 29 in an alternative embodiment of a medical imaging system according to the invention. Apart from the bridge-shaped carrier 77, said alternative embodiment is substantially identical to the medical imaging system as shown in Fig. 1. The bridge-shaped carrier 77 comprises a horizontal plate-shaped supporting member 79, on which the second guide member 29 is mounted, a first substantially vertical leg 81, which is situated at the front side 23 of the lower coil system housing 3, and a second substantially vertical leg 83, which is situated at the back side of the lower coil system housing 3 opposite to the front side 23. The first leg 81 is mounted to and supported by the second docking member 21, and the second leg 83 is supported and guided by rolling members 85 over a guiding surface 87, which extends in the second horizontal direction Y and is provided on the foot 75 of the frame 1. Alternatively, the rolling members 85 may be guided over the floor surface.

The medical imaging system according to the invention described before is a magnetic resonance imaging system of the so-called open type. It is noted that the invention also includes magnetic resonance imaging systems of the so-called closed type having a relatively narrow cylindrical examination volume. Although such a narrow examination volume allows only limited displacements of the patient support member in the second horizontal direction Y, the invention is still advantageous for closed type systems having two or more than two mobile carriers, each with a separate patient support member, which can alternately be docked to the frame. It is further noted that the invention also includes medical imaging systems of a different kind than magnetic resonance imaging systems such as, for example, X-ray or CT imaging systems.

It is further noted that the invention also includes embodiments in which the second displacement device for displacing the second docking member 21 in the second horizontal direction Y is of a different kind than the displacement device 33 shown in Fig. 4. The second displacement device may, for example, be provided with a toothed bar, mounted to the frame 1, and a toothed wheel co-operating with the toothed bar and driven by an electric motor mounted to the second docking member 21. It is finally noted that the invention also includes embodiments in which the docking members 19 and 21 are positioned in a different vertical position than the vertical position close to the floor surface shown in Fig.1.

## Claims

1. Medical imaging system comprising a frame (1) accommodating an imaging device and an examination volume (7), the system further comprising a patient support member (13) provided on a mobile carrier (15), having a first displacement device (31) for displacing the patient support member along a first guide member (25) on the carrier (15) in the longitudinal horizontal direction of the mobile carrier into and out of the examination volume (7) of the imaging device, and having a second displacement device (33) mounted to the frame for displacing the patient support member in a horizontal direction transverse to the longitudinal horizontal direction of the mobile carrier, the mobile carrier (15) comprises a first docking member (19) and the second displacement device (33) comprises a second docking member (21), the first and second docking members are provided for docking to each other, **characterized in that** the second docking member (21) is mounted to a second guide member (29) provided in the examination volume and extending in the longitudinal horizontal direction of and in registration with the first guide member (25) for supporting and guiding the patient support member in the examination volume (7).

2. Medical imaging system as claimed in claim 1, **characterized in that** the second displacement device (33) comprises a linear guide member (35) extending in the transverse horizontal direction and which is mounted to the frame (1), and the second docking element (21) comprises a guide element (39) mechanically co-operating with the linear guide element (35) for displacing the second docking element (21).

3. Medical imaging system as claimed in claim 1 or 2, **characterized in that** the system is a magnetic resonance imaging system and the frame (1) comprises a lower coil system housing (3) and an upper coil system housing (5) between which the examination volume (7) is present, the second guide member is provided on a bridge-shaped carrier (57) which bridges over the lower coil system housing (3) and comprises a first leg (59), which is mounted to and supported by the second docking member (21), and a second leg (63), which is present at a side of the lower coil system housing (3) opposite to a side where the second docking member (21) is present and which is connected to the first leg by means of a connecting member (71) extending underneath the lower coil system housing.

4. Medical imaging system as claimed in claim 1 or 2, **characterized in that** the system is a magnetic resonance imaging system and the frame (1) comprises a lower coil system housing (3) and an upper coil system housing (5) between which the examination volume is present, the second guide member (29) is provided on a bridge-shaped carrier (77) which bridges over the lower coil system housing (3) and comprises a first leg (81), which is mounted to and supported by the second docking member (21), and a second leg (83), which is supported by rolling members (85) at a side of the lower coil system housing opposite a side where the second docking member is present.

5. Medical imaging system as claimed in claim 4, **characterized in that** the bridge-shaped carrier (77) comprises a plate supporting member (79), on which the second guide member (29) is mounted.

## Patentansprüche

1. Medizinisches Bildgebungssystem mit einem die Bildgebungsvorrichtung und ein Untersuchungsvolumen (7) aufnehmenden Rahmen (1), wobei das System weiterhin Folgendes umfasst: ein Patientenauflageelement (13) auf einem mobilen Träger (15), mit einer ersten Verschiebungsvorrichtung (31) zum Verschieben des Patientenauflageelements entlang eines ersten Führungselements (25) auf dem Träger (15) in der horizontalen Längsrichtung des mobilen Trägers in das Untersuchungsvolumen (7) der Bildgebungsvorrichtung hinein und aus dem Untersuchungsvolumen heraus, und mit einer an dem Rahmen angebrachten zweiten Verschiebungsvorrichtung (33) zum Verschieben des Patientenauflageelements in einer horizontalen Richtung quer zu der horizontalen Längsrichtung des mobilen Trägers, wobei der mobile Träger (15) ein erstes Andockelement (19) umfasst und die zweite Verschiebungsvorrichtung (33) ein zweites Andockelement (21) umfasst, wobei das erste und das zweite Andockelement zum Andocken aneinander vorgesehen sind, **dadurch gekennzeichnet, dass** das zweite Andockelement (21) an einem zweiten Führungselement (29) angebracht ist, das in dem Untersuchungsvolumen vorgesehen ist und sich in horizontaler Längsrichtung des ersten Führungselements (25) und in Übereinstimmung hiermit erstreckt, um das Patientenauflageelement in dem Untersuchungsvolumen (7) zu stützen und zu führen.

2. Medizinisches Bildgebungssystem nach Anspruch 1, **dadurch gekennzeichnet, dass** die zweite Verschiebungsvorrichtung (33) ein lineares Führungselement (35) umfasst, das in horizontaler Querrichtung verläuft und an dem Rahmen (1) angebracht ist, und das zweite Andockelement (21) ein Führungselement (39) umfasst, das mechanisch mit dem linearen Führungselement (35) zusammenwirkt, um das zweite Andockelement (21) zu verschieben.

3. Medizinisches Bildgebungssystem nach Anspruch 1 oder 2, **dadurch gekennzeichnet, dass** das System ein Magnetresonanz-Bildgebungssystem ist und der Rahmen (1) ein unteres Spulensystemgehäuse (3) und ein oberes Spulensystemgehäuse (5) umfasst, zwischen denen sich das Untersuchungsvolumen (7) befindet, wobei das zweite Führungselement auf einem brückenförmigen Träger (57) vorgesehen ist, der das untere Spulensystemgehäuse (3) überspannt und ein erstes Bein (59) umfasst, das an dem zweiten Andockelement (21) montiert ist und von diesem gestützt wird, und ein zweites Bein (63) umfasst, das sich an einer Seite des unteren Spulensystemgehäuses (3) gegenüber einer Seite befindet, an der sich das zweite Andockelement (21) befindet, und das mittels eines unterhalb des unteren Spulensystemgehäuses verlaufenden Verbindungselements (71) mit dem ersten Bein verbunden ist.

4. Medizinisches Bildgebungssystem nach Anspruch 1 oder 2, **dadurch gekennzeichnet, dass** das System ein Magnetresonanz-Bildgebungssystem ist und der Rahmen (1) ein unteres Spulensystemgehäuse (3) und ein oberes Spulensystemgehäuse (5) umfasst, zwischen denen sich das Untersuchungsvolumen befindet, wobei das zweite Führungselement (29) auf einem brückenförmigen Träger (77) vorgesehen ist, der das untere Spulensystemgehäuse überbrückt (3) und ein erstes Bein (81) umfasst, das an dem zweiten Andockelement (21) montiert ist und von diesem gestützt wird, und ein zweites Bein (83) umfasst, das durch Rollelemente (85) an einer Seite des unteren Spulensystemgehäuses gegenüber einer Seite, an der sich das zweite Andockelement befindet, gestützt wird.

5. Medizinisches Bildgebungssystem nach Anspruch 4, **dadurch gekennzeichnet, dass** der brückenförmige Träger (77) ein Plattenstützelement (79) umfasst, auf dem das zweite Führungselement (29) montiert ist.

## Revendications

1. Système d'imagerie médicale comprenant un bâti (1) dans lequel sont logés un dispositif d'imagerie et un volume d'examen (7), le système comprenant un élément de support de patient (13) prévu sur un chariot mobile (15), comportant un premier dispositif de déplacement (31) pour déplacer l'élément de support de patient le long d'un premier organe de guidage (25) sur le chariot (15) dans la direction horizontale longitudinale du chariot mobile dans et hors du volume d'examen (7) du dispositif d'imagerie, et comportant un second dispositif de déplacement (33) monté sur le bâti pour déplacer l'élément de support de patient dans une direction horizontale transversale à la direction horizontale longitudinale du chariot mobile, le chariot mobile (15) comporte un premier élément d'arrimage (19) et le second dispositif de déplacement (33) comporte un second élément d'arrimage (21), le premier et le second élément d'arrimage sont prévus pour s'arrimer l'un à l'autre, **caractérisé en ce que** le second élément d'arrimage (21) est monté sur un second organe de guidage (29) prévu dans le volume d'examen et s'étendant dans la direction horizontale longitudinale du premier organe de guidage (25) et en coïncidence avec celui-ci pour supporter et guider l'élément de support de patient dans le volume d'examen (7).

2. Système d'imagerie médicale suivant la revendication 1, **caractérisé en ce que** le second dispositif de déplacement (33) comprend un organe de guidage linéaire (35) qui s'étend dans la direction horizontale transversale et qui est monté sur le bâti (1), et le second élément d'arrimage (21) comprend un élément de guidage (39) coopérant mécaniquement avec l'organe de guidage linéaire (35) afin de déplacer le second élément d'arrimage (21).

3. Système d'imagerie médicale suivant la revendication 1 ou 2, **caractérisé en ce que** le système est un système d'imagerie à résonance magnétique et le bâti (1) comprend un boîtier de système de bobines inférieur (3) et un boîtier de système de bobines supérieur (5) entre lesquels le volume d'examen (7) est présent, le second organe de guidage est prévu sur un support en forme de pont (57) qui s'étend au-dessus du boîtier de système de bobines inférieur (3) et comprend un premier pied (59), qui est monté sur le second élément d'arrimage (21) et est supporté par celui-ci, et un second pied (63) qui est présent d'un côté du boîtier de système de bobines inférieur (3) opposé à un côté où le second élément d'arrimage (21) est présent et qui est relié au premier pied au moyen d'une entretoise (71) qui s'étend en dessous du boîtier du système de bobines inférieur.

4. Système d'imagerie médicale suivant la revendication 1 ou 2, **caractérisé en ce que** le système est un système d'imagerie à résonance magnétique et le bâti (1) comprend un boîtier de système de bobines inférieur (3) et un boîtier de système de bobines supérieur (5) entre lesquels le volume d'examen (7) est présent, le second organe de guidage (29) est prévu sur un support en forme de pont (57) qui s'étend au-dessus du boîtier de système de bobines inférieur (3) et comprend un premier pied (81), qui est monté sur le second élément d'arrimage (21) et est supporté par celui-ci, et un second pied (83) qui est supporté par des organes roulants (85) au niveau d'un côté du boîtier de système de bobines inférieur opposé à un côté où le second élément d'arrimage est présent.

5. Système d'imagerie médicale suivant la revendication 4, **caractérisé en ce que** le support en forme de pont (77) comprend un élément de support en forme de plaque (79) sur lequel le second organe de guidage (29) est monté.
